# EUROPEAN PATENT APPLICATION

(11) **EP 3 828 258 A1**
(43) Date of publication of application: **02.06.2021**
(21) Application number: 19842316.2
(22) Date of filing: 19.07.2019
(51) Int. Cl.: C12M 1/00, C12N 15/87

(54) **BUBBLE JETTING METHOD, POWER SUPPLY DEVICE, AND DEVICE FOR JETTING BUBBLES**

(30) Priority: 23.07.2018 JP 2018138069
(71) Applicant: Bex Co., Ltd., Tokyo 173-0004 (JP)
(72) Inventor: MAKABE So, Tokyo 173-0004 (JP); WATANABE Hiromichi, Tokyo 173-0004 (JP); MORIIZUMI Yasuhiro, Tokyo 173-0004 (JP); MORIIZUMI Toshiyuki, Fujimino-shi, Saitama 356-0050 (JP)
(74) Representative: Heyerhoff Geiger & Partner Patentanwälte PartGmbB
(86) International application number: PCT/JP2019/028396
(87) International publication number: WO 2020/022198

(57) **Abstract**

Provided are: a bubble jetting method based on a new principle; a power-supply device for implementing said bubble jetting method; and a device for jetting bubbles. The present invention is capable of jetting bubbles by means of this method for jetting bubbles to a conductor, the method comprising: a step for bringing a bubble generation electrode into contact with the conductor; a step for bringing a counter electrode into contact with the conductor or an object to be processed; and a step for applying a negative voltage to the bubble generation electrode, wherein a conductor material is exposed at least at the leading end of the bubble generation electrode.

## Description

### [Technical Field]

The present disclosure relates to a bubble ejection method, a power supply device, and a bubble ejecting apparatus. In particular, the present disclosure relates to a bubble ejection method that can continuously eject bubbles into a conductor in accordance with a novel bubble ejection principle, a power supply device used for performing the bubble ejection method, and a bubble ejecting apparatus.

### [Background Art]

Due to progress of biotechnology in recent years, there is a growing demand for local processing of a cell or the like, such as processing to perforate a membrane or a wall of a cell and remove a nucleus from the cell or introduce a nucleic acid substance such as DNA to the cell. As a local processing technology (hereafter, also referred to as "local ablation method"), methods using a contact processing technology using a probe such as an electrical scalpel, a contactless ablation technology using laser or the like, or the like are widely known.

Further, as a local physical injection technology for introducing a nucleic acid substance or the like to a cell or the like (hereafter, also referred to as "local injection method"), an electroporation, a sonoporation technology using ultrasonic waves, a particle gun method, and the like are widely known.

Furthermore, not only local physical injection technologies for introducing a nucleic acid substance or the like to a cell or the like, such as a contact processing technology using a probe such as an electrical scalpel, a contactless ablation technology using laser or the like, an electroporation as described above but also a local ablation method using a bubble ejection member and a local ablation method and a local injection method using a vapor-liquid ejection member are known (see Patent Literature 1).

A bubble ejection member disclosed in Patent Literature 1 described above includes: (1) a core formed of a conductive material; (2) a shell portion formed of an insulating material, covering the core, and including a portion extending from the tip of the core; (3) a space formed between the extending portion of the shell portion and the tip of the core; and (4) a bubble ejection port formed at the tip of the extending portion (on the opposite side of the core in the space). Further, it is disclosed that, when ejecting a bubble by using the bubble ejection member, (5) first, by immersing at least the bubble ejection port of the bubble ejection member and a counter electrode in a solution, (6) next, by applying a high frequency voltage to the core of the bubble ejection member and the counter electrode, resulting in that (7) a bubble occurs in the space, and the bubble occurring in the space is then discharged so as to be pulled and cut from the bubble ejection port, and (8) by continuously emitting the discharged bubbles to a processing target, it is possible to cut (perform local ablation on) the processing target.

Further, Patent Literature 1 discloses that (9) by providing an outer shell portion outside the shell portion of the bubble ejection member so as to have a space with the shell portion, (10) by introducing, to the space, a solution in which an injection substance is dissolved and/or dispersed and thereby enabling generation of bubbles on which the solution in which an injection substance is dissolved and/or dispersed is absorbed to the interface, and (11) by continuously emitting the bubbles to the processing target, it is possible to cut the processing target and inject the injection substance contained in the solution covering the bubbles to the processing target.

As a technology similar to the above disclosure of Patent Literature 1, a bubble ejection chip in which a bubble ejection portion is formed such that a bubble ejection port is opened above a substrate is known (see Patent Literature 2). The bubble ejection chip disclosed in Patent Literature 2 includes (1) at least a substrate, a current conduction portion, and a bubble ejection portion, and it is disclosed (2) that the current conduction portion is formed above the substrate, (3) that the bubble ejection portion includes an electrode formed of a conductive material, a shell portion formed of an insulating photosensitive resin, and an extending portion extending from the shell portion, the shell portion covers the periphery of the electrode, and the extending portion extends from the tip of the electrode, and furthermore, the bubble ejection portion includes a space formed between the extending portion and the tip of the electrode, and (4) that the electrode of the bubble ejection portion is formed above the current conduction portion.

### [Citation List]

### [Patent Literature]

Patent Literature 1: International Publication No. WO 2013/129657
Patent Literature 2: International Publication No. WO 2017/069085

### [Summary of Invention]

### [Technical Problem]

As described above, in both of the bubble ejection member disclosed in Patent Literature 1 and the bubble ejection portion disclosed in Patent Literature 2, the periphery of the core (electrode) formed of a conductive material is covered with the shell portion formed of an insulating material, and furthermore, a space is provided by using the extending portion extending from the shell portion and the core (electrode). Further, since a bubble is first generated in the space and then ejected from the bubble ejection port, the bubble ejection member and the bubble ejection portion have the extending portion extending from the shell portion as an essential component.

However, it is required to heat and cut off an insulating material such as glass to form the extending portion disclosed in Patent Literature 1. Further, it is required to form the extending portion disclosed in Patent Literature 2 with a photosensitive resin by using a photolithography technology. Thus, there is a problem of increased complexity of the manufacturing process of the bubble ejection member and the bubble ejection chip including the bubble ejection portion.

The disclosure in the present specification has been made in order to solve the problems described above, and according to a thorough study, it has been newly found that (1) by applying a negative voltage to a bubble generating electrode formed of a conductive material, (2) it is possible to eject a bubble into a conductor from the tip part of the bubble generating electrode with a state where the conductive material is exposed, in other words, without forming the extending portion and the space disclosed in Patent Literatures 1 and 2 in the tip part of the bubble generating electrode.

That is, the objective of the present disclosure is to provide a bubble ejection method based on a novel principle, a power supply device for performing the bubble ejection method, and a bubble ejecting apparatus.

### [Solution to Problem]

The present disclosure relates to a bubble ejection method, a power supply device, and a bubble ejecting apparatus illustrated below.

(1) A bubble ejection method into a conductor, the bubble ejection method comprising steps of:
   contacting a bubble generating electrode to the conductor;
   contacting a counter electrode to the conductor or a processing target; and
   applying a negative voltage to the bubble generating electrode,
   wherein a conductive material is exposed on at least a tip part of the bubble generating electrode.
(2) The bubble ejection method according to (1) above, wherein the step of applying the negative voltage to the bubble generating electrode
   applies a pulsed voltage, and
   starts application to the bubble generating electrode from a negative voltage.
(3) The bubble ejection method according to (2) above further comprising a step of:
   applying a positive voltage continuously to the step of applying the negative voltage to the bubble generating electrode.
(4) The bubble ejection method according to (1) above,
   wherein the step of applying the negative voltage to the bubble generating electrode applies an alternating voltage, and
   wherein the alternating voltage has an amplitude on a negative voltage side.
(5) The bubble ejection method according to any one of (1) to (4) above, wherein a part other than the tip part, on which the conductive material is exposed, of the bubble generating electrode is covered with an insulating material.
(6) The bubble ejection method according to any one of (1) to (5) above, wherein a surface area of the bubble generating electrode in contact with the conductor is smaller than a surface area of the counter electrode in contact with the conductor or a processing target.
(7) The bubble ejection method according to any one of (1) to (6) above, wherein the tip part of the bubble generating electrode has a sharp-pointed shape.
(8) A power supply device comprising a control unit used for controlling a voltage to be applied,
   wherein the control unit performs control so as to start application from a negative pulsed voltage.
(9) A power supply device comprising a control unit used for controlling a voltage to be applied,
   wherein the control unit performs control so as to apply an alternating voltage having an amplitude on a negative voltage side.
(10) A bubble ejecting apparatus comprising at least a bubble generating electrode and the power supply device according to (8) or (9) above.

### [Advantageous Effect of Invention]

The bubble ejection method disclosed in the present specification can eject a bubble into a conductor by using the bubble generating electrode on which a conductive material is exposed. It is therefore possible to provide a bubble ejection method with a novel principle. Further, the power supply device can be suitably used for the bubble ejection method with the novel principle. Furthermore, by combining the power supply device and the bubble generating electrode, it is possible to provide a bubble ejecting apparatus with the novel principle.

### [Brief Description of Drawings]

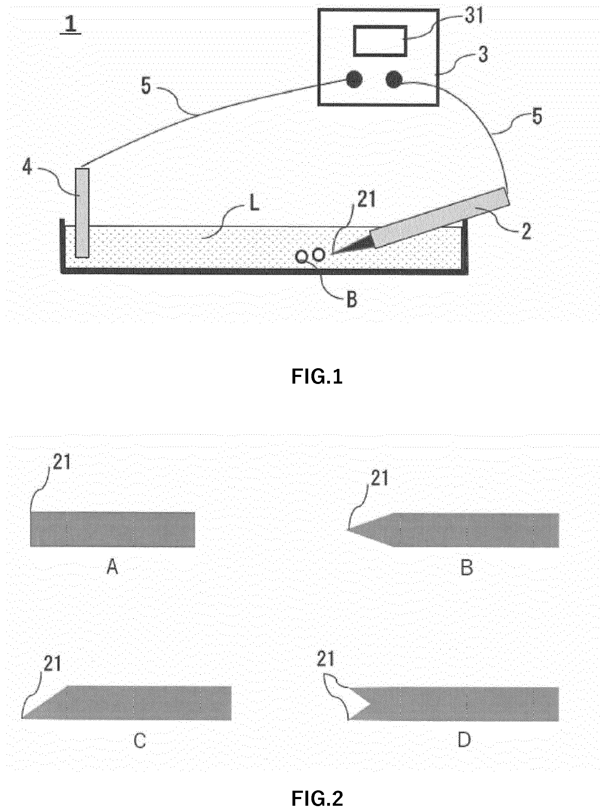

[FIG. 1] FIG. 1 is a diagram illustrating an overview of an embodiment of a bubble ejecting apparatus.
[FIG. 2] FIG. 2 is a diagram illustrating an example of a tip part 21 of a bubble generating electrode 2.

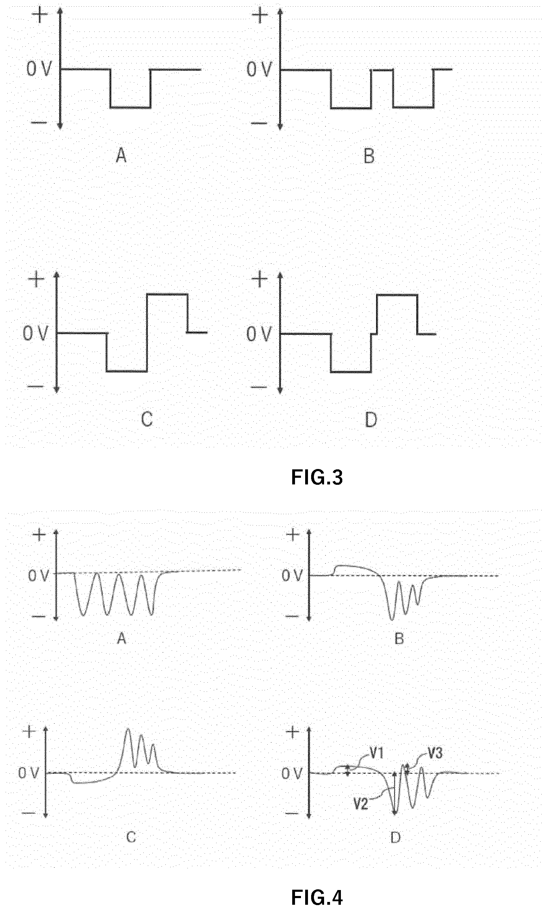

[FIG. 3] FIG. 3 is a diagram illustrating an example of a pulsed voltage output by a novel power supply device 3.
[FIG. 4] FIG. 4 is a diagram illustrating an example of an alternating voltage output by the novel power supply device 3.

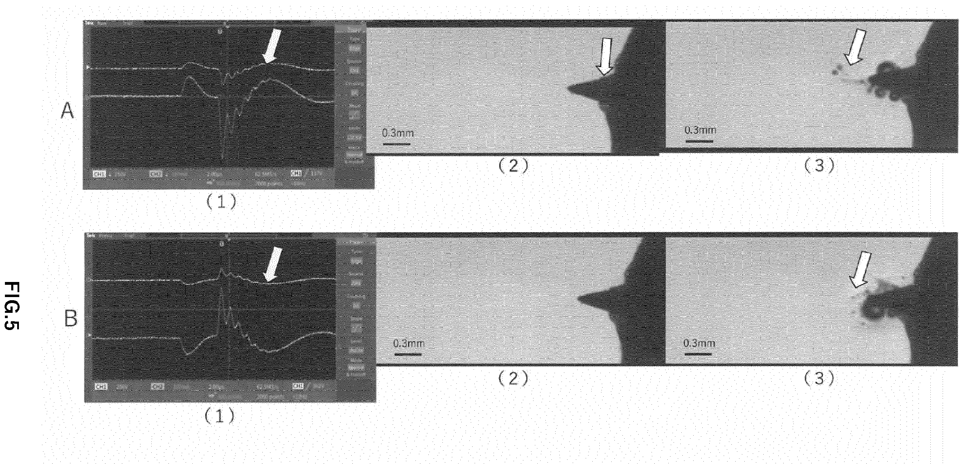

[FIG. 5] FIG. 5A illustrates photographs substitute for drawing, which are (1) a chart of a voltage applied in Example 1, (2) a photograph of the tip part of the bubble generating electrode before the voltage is applied, and (3) a photograph of the tip part of the bubble generating electrode after the voltage is applied. FIG. 5B illustrates photographs substitute for drawing, which are (1) a chart of a voltage applied in Example 2, (2) a photograph of the tip part of the bubble generating electrode before the voltage is applied, and (3) a photograph of the tip part of the bubble generating electrode after the voltage is applied.

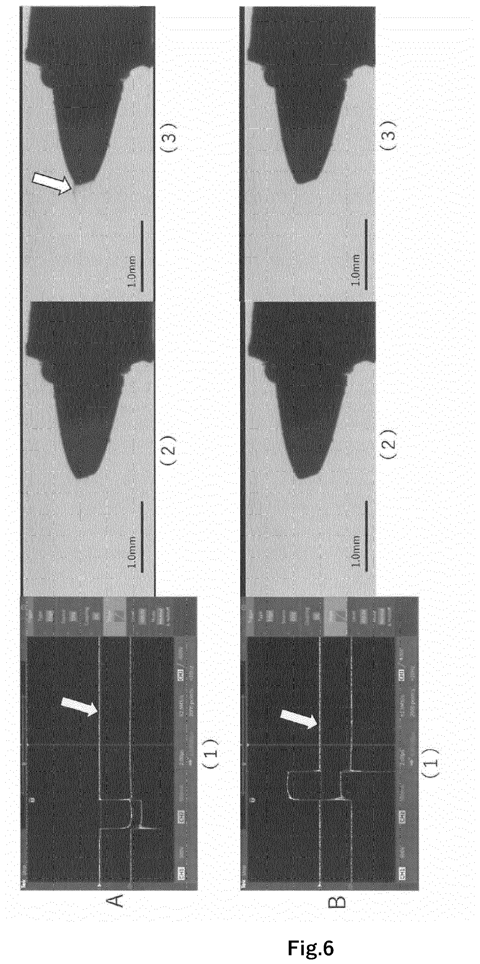

[FIG. 6] FIG. 6A illustrates photographs substitute for drawing, which are (1) a chart of a voltage applied in Example 3, (2) a photograph of the tip part of the bubble generating electrode before the voltage is applied, and (3) a photograph of the tip part of the bubble generating electrode after the voltage is applied. FIG. 6B illustrates photographs substitute for drawing, which are (1) a chart of a voltage applied in Comparative example 1, (2) a photograph of the tip part of the bubble generating electrode before the voltage is applied, and (3) a photograph of the tip part of the bubble generating electrode after the voltage is applied.

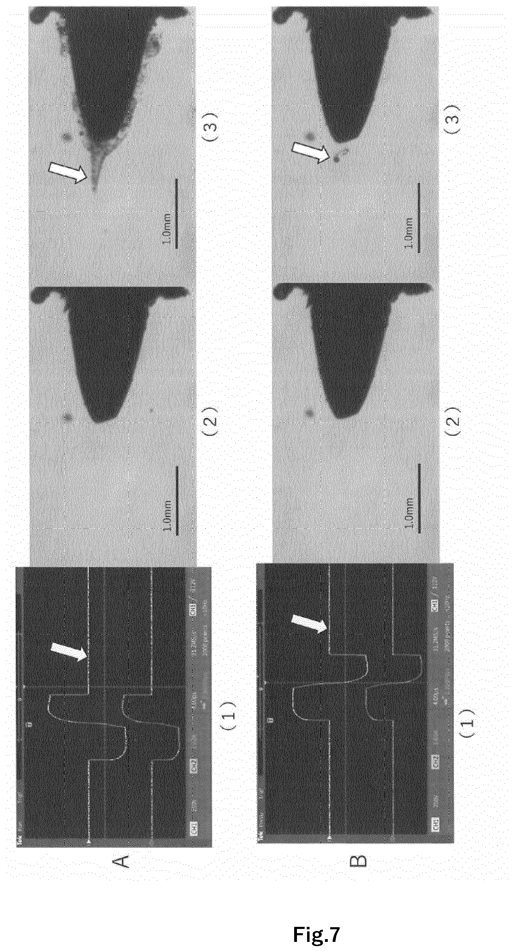

[FIG. 7] FIG. 7A illustrates photographs substitute for drawing, which are (1) a chart of a voltage applied in Example 4, (2) a photograph of the tip part of the bubble generating electrode before the voltage is applied, and (3) a photograph of the tip part of the bubble generating electrode after the voltage is applied. FIG. 7B illustrates photographs substitute for drawing, which are (1) a chart of a voltage applied in Example 5, (2) a photograph of the tip part of the bubble generating electrode before the voltage is applied, and (3) a photograph of the tip part of the bubble generating electrode after the voltage is applied.

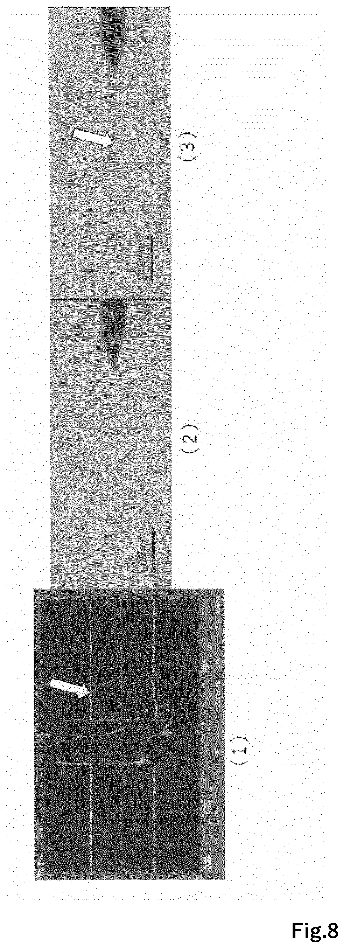

[FIG. 8] FIG. 8 illustrates photographs substitute for drawing, which are (1) a chart of a voltage applied in Example 6, (2) a photograph of the tip part of the bubble generating electrode before the voltage is applied, and (3) a photograph of the tip part of the bubble generating electrode after the voltage is applied.

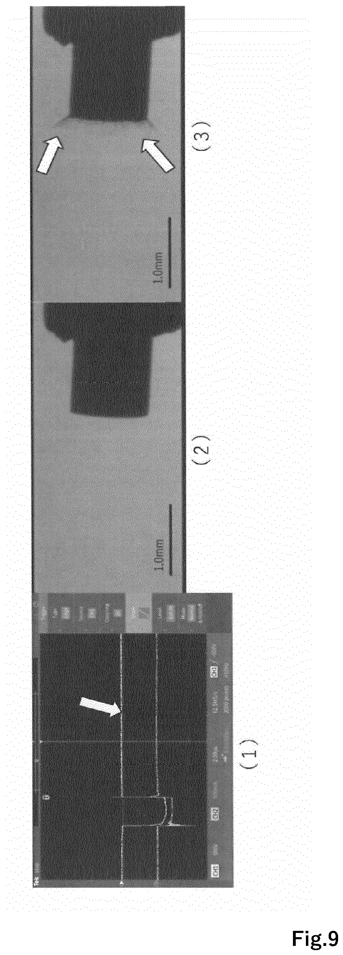

[FIG. 9] FIG. 9 illustrates photographs substitute for drawing, which are (1) a chart of a voltage applied in Example 7, (2) a photograph of the tip part of the bubble generating electrode before the voltage is applied, and (3) a photograph of the tip part of the bubble generating electrode after the voltage is applied.

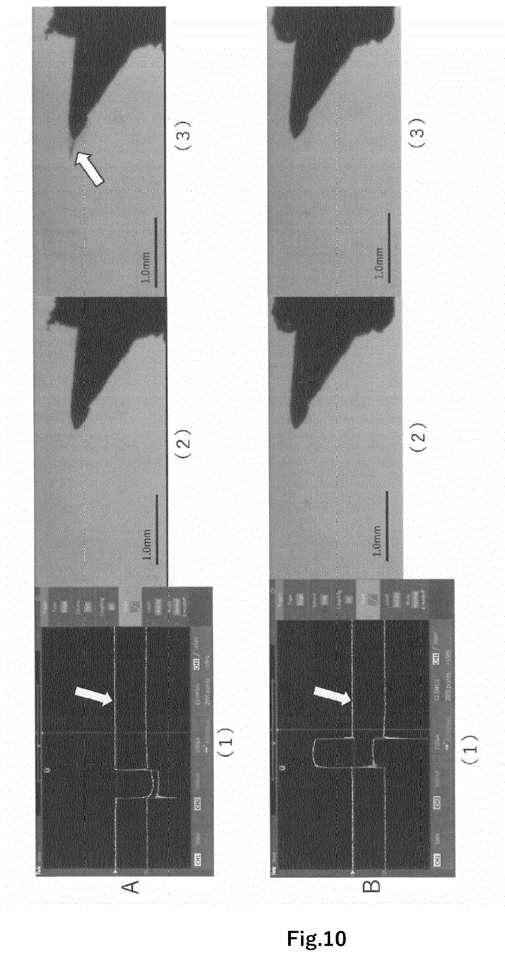

[FIG. 10] FIG. 10A illustrates photographs substitute for drawing, which are (1) a chart of a voltage applied in Example 8, (2) a photograph of the tip part of the bubble generating electrode before the voltage is applied, and (3) a photograph of the tip part of the bubble generating electrode after the voltage is applied. FIG. 10B illustrates photographs substitute for drawing, which are (1) a chart of a voltage applied in Comparative example 2, (2) a photograph of the tip part of the bubble generating electrode before the voltage is applied, and (3) a photograph of the tip part of the bubble generating electrode after the voltage is applied.

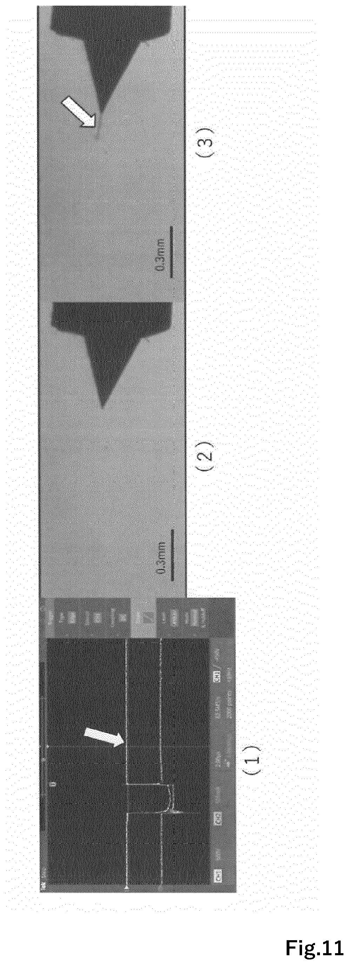

[FIG. 11] FIG. 11 illustrates photographs substitute for drawing, which are (1) a chart of a voltage applied in Example 9, (2) a photograph of the tip part of the bubble generating electrode before the voltage is applied, and (2) a photograph of the tip part of the bubble generating electrode after the voltage is applied.

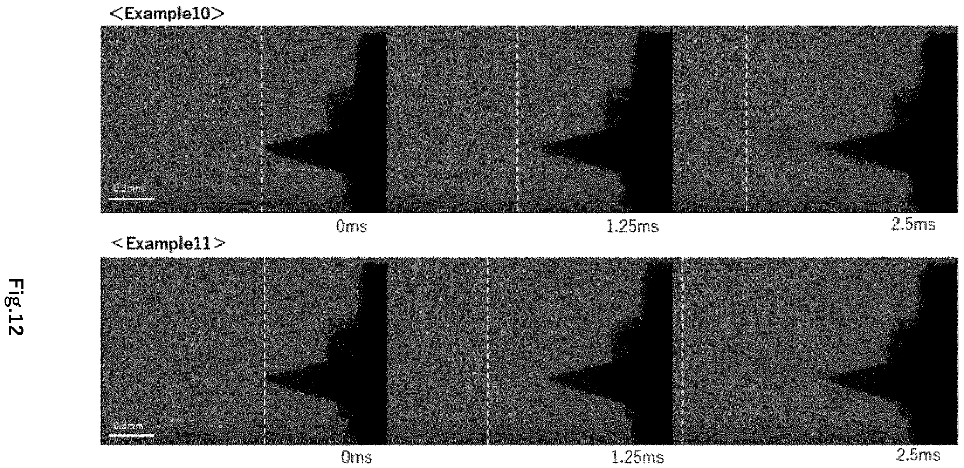

[FIG. 12] FIG. 12 illustrates photographs substitute for drawing, which are photographs in which frames of a high-speed camera is analyzed for comparing positions of bubbles ejected in Examples 10 and 11.

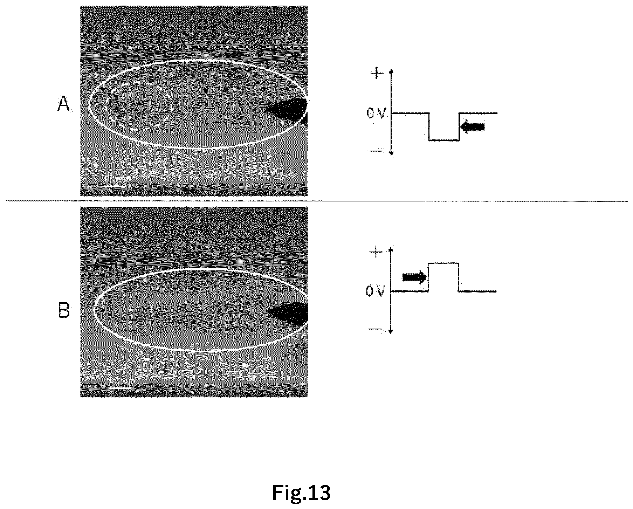

[FIG. 13] FIG. 13A illustrates a photograph substitute for drawing, which is a photograph of the tip part of the bubble generating electrode after a voltage is applied in Example 12. FIG. 13B illustrates a photograph substitute for drawing, which is a photograph of the tip part of the bubble generating electrode after a voltage is applied in Comparative example 3.

### [Description of Embodiments]

A bubble ejection method, a power supply device, and a bubble ejecting apparatus will be described below in detail with reference to the drawings.

### [Embodiment of Bubble ejecting apparatus]

FIG. 1 is a diagram illustrating an overview of an embodiment of the bubble ejecting apparatus. The bubble ejecting apparatus 1 includes at least a bubble generating electrode 2 and a power supply device 3. A counter electrode 4 may be a member forming the bubble ejecting apparatus 1 or may be a separate member from the bubble ejecting apparatus 1 and be prepared where necessary. Further, the bubble generating electrode 2 and the power supply device 3 may be connected to each other in advance by an electric cable 5 or may be connected where necessary, and the counter electrode 4 and the power supply device 3 may be connected to each other in advance by an electric cable 5 or may be connected where necessary.

The bubble generating electrode 2 is formed of a conductive material, and the conductive material is exposed on a tip part 21. The conductive material is not particularly limited as long as it conducts electricity and can be used as an electrode, and the conductive material is preferably a metal, which may be, for example, gold, silver, copper, iron, aluminum, platinum, tungsten, or the like or an alloy in which tin, magnesium, chromium, nickel, zirconium, silicon, iridium, or the like is added to the above metal, for example, stainless or the like. Further, carbon or the like may be used other than a metal.

The conductive material is exposed on at least the tip part of the bubble generating electrode 2. In other words, the bubble generating electrode 2 may be entirely formed of the conductive material, or a portion other than the tip part 21 may be covered with an insulating material. Note that, in the present specification, "tip part" means the end of the bubble generating electrode 2 on the side in contact with a conductor L. The bubble ejection method disclosed in the present specification is to concentrate a negative electric field on the tip of the bubble generating electrode 2 to eject a bubble. It is therefore preferable that the tip part 21 be significantly small in order to facilitate concentration of an electric field. If the tip part 21 is covered with an insulating material, the conductive material of the tip part 21 can be exposed by, for example, after producing an electrode body with a conductive material, first, immersing the electrode body in an insulating material, applying an insulating material to the electrode body, or depositing an insulating material on the electrode body to cover the periphery of the electrode body, and then removing the insulating material on the tip part 21 of the electrode body. Further, the electrode may be fitted into a tube formed of an insulating material so that the tip part 21 is exposed.

The insulating material is not particularly limited as long as it insulates electricity and may be, for example, an inorganic-based insulating material such as glass, mica, quartz, silicon nitride, silicon oxide, ceramic, alumina, or the like, a rubber material such as silicone rubber, an ethylene propylene rubber, or the like, or an insulating resin such as an ethylene-vinyl acetate copolymer resin, a silane modified olefin resin, an epoxy resin, a polyester resin, a vinyl chloride-based resin, an acrylic resin, a melamine resin, a phenolic resin, a polyurethane resin, a polystyrene-based resin, a fluorine-based resin, a silicon-based resin, a polysulfide-based resin, a polyamide resin, a polyimide resin, polyethylene, polypropylene, a cellulose-based resin, a UV curing resin, or the like.

FIG. 2 is a diagram illustrating an example of the tip part 21 of the bubble generating electrode 2. Since a smaller tip part 21 facilitates concentration of an electric field, it is preferable to produce the bubble generating electrode 2 such that the tip part 21 is small as much as possible. For example, as illustrated in FIG. 2A, it is possible to produce such a tip part 21 by producing a thin wire whose diameter is small as much as possible by using the conductive material described above and cutting the same. Further, as illustrated in FIG. 2B and FIG. 2C, such a tip part 21 may be formed by first producing a thin wire and then polishing the tip to form a sharp-pointed shape toward the tip part 21 of the bubble generating electrode 2. Note that, when forming a sharp-pointed shape, a shape having no edge, such as a cone, is preferable rather than a shape having an edge, such as a pyramid. In other words, the cross section of the bubble generating electrode 2 is preferably formed of a curved surface having no corner, and a shape whose sectional area decreases as approaching the tip part 21 is preferable. Note that, although a single tip part 21 is formed as illustrated FIG. 2B and FIG. 2C, two or more tip parts 21 may be formed as illustrated FIG. 2D.

As illustrated in Examples and Comparative examples described later, the bubble ejection method disclosed in the present specification can eject a bubble from the tip of the bubble generating electrode 2 by applying a negative voltage to the bubble generating electrode 2. Therefore, the power supply device used for the bubble ejection method is not particularly limited as long as it can be applied with a negative voltage.

As illustrated in Examples and Comparative examples described later, as a voltage applied to the bubble generating electrode 2, it is preferable to initially apply a negative pulsed voltage or apply a voltage having an amplitude on the negative voltage side. However, since current flows from the positive side to the negative side, general commercial power supply devices are designed to apply a voltage from the positive side. Thus, the power supply device 3 disclosed in the present specification is produced so as to be able to initially apply a negative pulsed voltage or apply a voltage having an amplitude on the negative voltage side in accordance with program design. In the power supply device 3 illustrated in FIG. 1, a newly designed program is stored in a control unit 31. That is, the power supply device 3 disclosed in the embodiment of the bubble ejecting apparatus is a novel power supply device.

FIG. 3 is a diagram illustrating an example of a pulsed voltage (applied to the bubble generating electrode 2) output by the novel power supply device 3. FIG. 3A illustrates an example in which the negative pulsed voltage is applied once, and FIG. 3B illustrates an example in which the negative pulsed voltage is applied twice. Note that, in a case of multiple times of application, the number of times thereof is not particularly limited. Further, FIG. 3C illustrates an example in which a positive pulsed voltage is applied continuously to a step of applying the negative pulsed voltage. As illustrated in the Examples described later, in the bubble ejection method disclosed in the present specification, it is considered that (1) a bubble B is generated when a negative voltage is applied to the bubble generating electrode 2, (2) a flow of a conductor from the bubble generating electrode 2 to the counter electrode 4 is generated when a voltage that is positive relatively to the negative voltage is applied, and (3) the generated flow of the conductor pushes out the generated bubble B (the bubble B is ejected). Therefore, when the pulsed voltage is applied, as long as a negative voltage is initially applied, a positive voltage may be continuously applied. Note that "continuous" as used in the present specification means that the voltage may change from the negative to the positive directly as illustrated in FIG. 3C or that there may be a period of time when no voltage is applied (a period of time of 0 V) in application of a voltage changing from negative to positive, as illustrated in FIG. 3D as long as ejection of the generated bubble B is not affected. Further, when "application of a negative voltage and then application of a positive voltage" illustrated in FIG. 3C and FIG. 3D is defined as one set, the set may be repeated for multiple times.

FIGs. 4A, 4B, and 4D are diagrams illustrating examples of an alternating voltage (applied to the bubble generating electrode 2) output by the novel power supply device 3. When an alternating voltage is applied to the bubble generating electrode 2, any voltage having an amplitude on the negative voltage side can be applied. For example, as illustrated in FIG. 4A, there is an example in which the amplitude is always on the negative voltage side. Further, as illustrated in FIG. 4B, after a positive voltage is applied once at the initial voltage application, an alternating voltage in which a negative voltage is applied while the amplitude is on the negative voltage side may be employed. As illustrated in FIG. 4C, however, in a case of an alternating voltage in which a positive voltage is applied while the amplitude is on the positive voltage side even after a negative voltage is initially applied, although bubbles are ejected, the amount of the bubbles ejected from the bubble generating electrode 2 is reduced. Therefore, "a voltage having an amplitude on the negative voltage side" output by the novel power supply device 3 means not only that a whole amplitude occurs on the negative voltage side as illustrated in FIG. 4A but also that most of an amplitude occurs on the negative voltage side as illustrated in FIG. 4B and FIG. 4D. For example, it means a voltage whose absolute value (V2) of any peak on the negative voltage side is larger than the absolute values (V1, V3) of the positive components of the adjacent positive peaks, as illustrated in FIG. 4D.

The voltage applied to the bubble generating electrode 2, in other words, the voltage output by the power supply device 3 is not particularly limited as long as it can cause a bubble to be ejected from the bubble generating electrode 2. The voltage to be applied can be suitably set in accordance with the material, the size, or the shape of the tip part in which an electric field concentrates of the bubble generating electrode 2, and the type of the conductor contacted by the bubble generating electrode 2 and the conductor contacted by the counter electrode 4 or the type of a processing target. When the voltage output by the power supply device 3 is a pulsed voltage, the pulse width, the pulse interval when multiple pulses are applied, the output current, or the like are not particularly limited and can be suitably set as long as a bubble can be ejected from the bubble generating electrode 2. Further, when the voltage output by the power supply device 3 is an alternating voltage, the frequency, the output current, or the like are not particularly limited and can be suitably set as long as a bubble can be ejected from the bubble generating electrode 2.

The counter electrode 4 is not particularly limited as long as it conducts electricity, and the same material as that of the bubble generating electrode 2 can be used. The materials of the bubble generating electrode 2 and the counter electrode 4 may be the same or may be different from each other. Further, the counter electrode 4 is not particularly limited in the shape or the like as long as it can contact the exposed conductive material to the conductor or a processing target, and any shape such as a liner shape, a plate-like shape, or the like can be employed. Note that, although it is preferable to reduce the size of the bubble generating electrode 2 so that an electric field concentrates therein, it is not necessary to reduce the size of the counter electrode 4. Therefore, the counter electrode 4 may be larger than the bubble generating electrode 2. In other words, the bubble generating electrode 2 is smaller in comparison between the surface area of the bubble generating electrode in contact with the conductor and the surface area of the counter electrode in contact with the conductor or the processing target.

### [Embodiment of Bubble Ejection Method]

Next, an embodiment of the bubble ejection method will be described with reference to FIG. 1. The embodiment of the bubble ejection method includes at least (1) a step of contacting the bubble generating electrode 2 to the conductor L, (2) a step of contacting the counter electrode to the conductor L or a processing target, and (3) a step of applying a negative voltage to the bubble generating electrode 2. In order to eject a bubble from the bubble generating electrode 2, it is required to cause the tip part 21 of the bubble generating electrode 2 to be in contact with (immersed in) the conductor L. On the other hand, the counter electrode 4 is only required to form a circuit with the bubble generating electrode 2. Therefore, as illustrated in FIG. 1, the counter electrode 4 and the bubble generating electrode 2 may be contacted to (immersed in) the conductor L, or when the processing target described later is conductive, at least a part of the processing target may be contacted to the conductor L, the bubble generating electrode 2 may be contacted to the conductor L, and the counter electrode 4 may be directly contacted with the processing target.

The conductor L is not particularly limited as long as the bubble generating electrode 2 and the counter electrode 4 can conduct current therein to eject a bubble and may be a liquid, a viscos liquid having a level of viscosity that does not prevent ejection of a bubble, a mixture of a liquid or a viscos liquid with a solid, or the like. The liquid may be, for example, water, a solution in which a salt such as KCl, NaCl₂, or the like is dissolved in water, or a buffer solution, a culture medium, or the like such as PBS used in the field of biology. The viscos liquid may be a viscos liquid in which a thickener is added to the above liquid or a biological component such as blood. The mixture of a liquid or a viscos liquid with a solid may be a biological tissue containing a large amount of a conductive body fluid.

By contacting the bubble generating electrode 2 to the conductor L, contacting the counter electrode 4 to the conductor L or the processing target, and then applying a negative voltage to the bubble generating electrode 2, it is possible to eject the bubble B from the tip part 21 of the bubble generating electrode 2 into the conductor L.

Further, as disclosed in Patent Literatures 1 and 2, by causing the bubble B ejected from the bubble generating electrode 2 by using the bubble ejection method disclosed in the present specification to collide with a processing target, it is possible to perform local ablation on the processing target. Further, when a liquid is used as the conductor L, it is possible to inject an injection substance to the processing target while performing local ablation on the processing target by dissolving a nucleic acid such as DNA or RNA, a protein, an amino acid, a soluble drug, or a gaseous injection substance such as nitrogen, helium, carbon dioxide, argon, or the like in the liquid in advance.

The processing target is not particularly limited as long as ablation can be performed thereon by using bubbles and may be an animal, a plant such as a leaf or a seed, an organism such as a microorganism, a tissue or a cell separated from such an organism, a protein, or the like. The cell may be a cell such as an animal cell such as a stem cell, a skin cell, a mucosal cell, a hepatocyte, an islet cell, a nerve cell, a chondrocyte, an endothelial cell, an epithelial cell, a bone cell, a muscle cell, an egg cell, or the like isolated from a tissue of a human or a non-human animal, a plant cell, an insect cell, or a microbial cell such as E. coli, a yeast, and a mold, or the like. Further, a resin, a metal, or the like may be used other than an organism.

For example, it is possible to perform ablation by using a liquid as a conductor, arranging a processing target between the bubble generating electrode 2 and the counter electrode 4 that are immersed in the liquid, and applying a negative voltage to the bubble generating electrode 2 to cause the ejected bubble to collide with the processing target. Note that, as described above, when the processing target is conductive, the counter electrode 4 may be in direct contact with the processing target. Further, when the processing target is a conductor (such as a biological tissue containing a large amount of conductive body fluid), it is possible to perform direct ablation on the processing target by contacting the bubble generating electrode 2 and the counter electrode 4 to the processing target and applying a negative voltage to the bubble generating electrode 2.

Although examples will be presented below to specifically describe each embodiment, these examples are provided only for the purpose of reference of a specific aspect thereof. These illustrations are not intended to limit or restrict the scope of the invention.

### [Examples]

### [Example of Applying Alternating Current to Bubble Generating Electrode]

### [Example 1]

### [Production of Bubble ejecting apparatus]

First, a thin wire was produced from stainless, abrasive machining was performed on the tip part of the thin wire, and thereby a bubble generating electrode having a substantial cone-shaped tip part was produced. FIG. 5A (2) illustrates a photograph of the tip part of the produced bubble generating electrode. Note that a spread portion on the right side in the photograph corresponds to an adhesive agent applied for preventing submergence. The length of the tip part (the downward length in the drawing sheet of a portion pointed by the arrow in FIG. 5A (2)) was approximately 0.3 mm. Next, a counter electrode was produced from an aluminum plate (5 mm by 10 mm by 0.044 mm). Next, Hyfrecator 2000 (ConMed Corp.) was used as a power supply device, and the power supply device was connected to the bubble generating electrode and the counter electrode by an electric cable. Note that the program was altered so that the voltage applied to the bubble generating electrode was initially a positive voltage and then the amplitude occurred on the negative voltage side.

### [Implementation of Bubble Ejection Method]

The tip part of the bubble generating electrode and the counter electrode of the produced bubble ejecting apparatus were immersed in phosphate buffered saline (PBS: Wako 163-25265 10×PBS(-) was diluted 10 times). Note that a volume of 5 mm by 5 mm by 0.044 mm of the counter electrode was immersed. Next, a Hi-mode (5 W) and continuous 300 sets with 5-cycle amplitudes being defined as one set were set as the conditions of Hyfrecator 2000 to apply the voltage. The state of the tip part of the bubble generating electrode was photographed in a condition of 1500 fps by using a high-speed camera (HAS-U2, by DITECT Co. Ltd.). The arrow of FIG. 5A (1) indicates a voltage change per set applied to the bubble generating electrode (in which the graph under the graph of voltage change pointed by the arrow represents a current change, and the same applies to the drawings below), FIG. 5A (2) is a photograph of the tip part of the bubble generating electrode before the voltage is applied, and FIG. 5A (3) is a photograph of the tip part of the bubble generating electrode after the voltage is applied in which the portion pointed by the arrow indicates ejected bubbles.

### [Example 2]

### [Production of Bubble Ejecting Apparatus]

Hyfrecator 2000 (ConMed Corp.) was used as the power supply device in the same manner, and the bubble ejecting apparatus was produced in the same procedure as that in Example 1 except that the voltage applied to the bubble generating electrode was changed so that initially a negative voltage was applied but then the amplitude occurred on the positive voltage side.

### [Implementation of Bubble Ejection Method]

A bubble ejection method was implemented in the same procedure as that in Example 1 except that the voltage applied to the bubble generating electrode had the amplitude occurring on the positive voltage side. The arrow of FIG. 5B (1) indicates a voltage change applied to the bubble generating electrode, FIG. 5B (2) is a photograph of the tip part of the bubble generating electrode before the voltage is applied, and FIG. 5B (3) is a photograph of the tip part of the bubble generating electrode after the voltage is applied.

When the voltage having an amplitude on the negative voltage side was applied to the bubble generating electrode as illustrated in FIG. 5A (1), ejection of bubbles from the tip part of the bubble generating electrode was observed as indicated by the arrow in FIG. 5A (3). On the other hand, when the negative voltage was initially applied to the bubble generating electrode and then the voltage having an amplitude on the positive voltage side was applied as illustrated in FIG. 5B (1), ejection of bubbles was observed at the tip part of the bubble generating electrode as illustrated in FIG. 5B (3), but the level of ejection of bubbles was lower than that in Example 1. From the above results, it was revealed that (1) when applying an alternating voltage to the bubble generating electrode, it is possible to eject a bubble from the tip of the bubble generating electrode by applying a negative voltage to the bubble generating electrode and (2) when conditions other than the voltage are the same, it is preferable to apply a voltage so as to cause the amplitude to occur on the negative voltage side.

### [Example and Comparative Example of Applying Pulsed Voltage to Bubble Generating Electrode]

### [Example 3]

### [Production of Bubble Ejecting Apparatus]

A bubble generating electrode whose tip part has substantially a cone shape that was cut at a small angle was produced in the same procedure as that in Example 1 except that copper instead of stainless was used. FIG. 6A (2) illustrates a photograph of the tip part of the produced bubble generating electrode. Further, the bubble ejecting apparatus was produced in the same procedure as that in Example 1 except that CFB 16 (BEX Co., Ltd.) instead of Hyfrecator 2000 (ConMed Corp.) was used as the power supply device, and the program was altered so that a negative pulsed voltage, in which the voltage falls from 0 V to the negative direction at a rate of approximately dV/dt = 10V/1µs and the voltage rises toward 0 V after pulse application is approximately dV/dt = 10V/1µs, can be applied to the bubble generating electrode.

### [Implementation of Bubble Ejection Method]

A bubble ejection method was performed in the same procedure as that in Example 1 except that 2 mM of KCl instead of PBS was used, and a pulsed voltage having a voltage of -1000 V, a pulse width of 3 µs, and a pulse interval of 150 µs was applied continuously for 50 times. The arrow of FIG. 6A (1) indicates a voltage change per pulse applied to the bubble generating electrode, FIG. 6A (2) is a photograph of the tip part of the bubble generating electrode before the voltage is applied, and FIG. 6A (3) is a photograph of the tip part of the bubble generating electrode after the voltage is applied in which the portion pointed by the arrow indicates ejected bubbles.

### [Comparative Example 1]

### [Production of Bubble Ejecting Apparatus]

A bubble ejecting apparatus was produced in the same procedure as that in Example 3 except that the power supply device of Example 3 was altered so as to be able to apply a positive voltage instead of a negative voltage.

### [Implementation of Bubble Ejection Method]

The bubble ejection method was performed in the same procedure as that in Example 3 except that the voltage applied to the bubble generating electrode was a positive pulsed voltage. The arrow of FIG. 6B (1) indicates a voltage change applied to the bubble generating electrode, FIG. 6B (2) is a photograph of the tip part of the bubble generating electrode before the voltage is applied, and FIG. 6B (3) is a photograph of the tip part of the bubble generating electrode after the voltage is applied.

When the negative pulsed voltage was applied to the bubble generating electrode as illustrated in FIG. 6A (1), ejection of bubbles from the tip part of the bubble generating electrode was observed as indicated by the arrow in FIG. 6A (3). On the other hand, when the positive pulsed voltage was applied to the bubble generating electrode as illustrated in FIG. 6B (1), no bubble was generated at the tip part of the bubble generating electrode as illustrated in FIG. 6B (3). From the above results, it was revealed that, when applying pulsed voltage to the bubble generating electrode, it is possible to eject a bubble from the tip part of the bubble generating electrode by applying a negative pulsed voltage.

### [Example 4]

Next, a bubble ejecting apparatus was produced and a bubble ejection method was performed in the same procedure as that in Example 3 except that the power supply device was altered so as to apply a positive voltage continuously from a negative pulsed voltage, 0.5×PBS instead of 2 mM of KCl was used, the positive voltage was +600 V, the negative voltage was -600 V, each pulse width of the positive voltage and the negative voltage was 6 µs, the pulse interval (the period of time from the end of application of a positive pulse to application of the next negative pulse) was 100 µs, and 50 sets were continuously applied with application of a negative voltage pulse and then a positive voltage pulse being defined as one set. The arrow of FIG. 7A (1) indicates a voltage change of one set of pulses applied to the bubble generating electrode, FIG. 7A (2) is a photograph of the tip part of the bubble generating electrode before the voltage is applied, and FIG. 7A (3) is a photograph of the tip part of the bubble generating electrode after the voltage is applied in which the portion pointed by the arrow indicates ejected bubbles.

### [Example 5]

A bubble ejecting apparatus was produced and a bubble ejection method was performed in the same procedure as that in Example 4 except that the power supply device was altered so as to apply a negative voltage continuously from a positive voltage. The arrow of FIG. 7B (1) indicates a voltage change applied to the bubble generating electrode, FIG. 7B (2) is a photograph of the tip part of the bubble generating electrode before the voltage is applied, and FIG. 7B (3) is a photograph of the tip part of the bubble generating electrode after the voltage is applied.

In Example 4 in which the negative to positive pulsed voltage was continuously applied to the bubble generating electrode as illustrated in FIG. 7A (1), ejection of a large number of bubbles from the tip part of the bubble generating electrode was observed as indicated by the arrow in FIG. 7A (3). On the other hand, when the positive to negative pulsed voltage was continuously applied to the bubble generating electrode as illustrated in FIG. 7B (1), ejection of bubbles from the tip part of the bubble generating electrode was observed as illustrated in FIG. 7B (3), but the level of the ejection of bubbles was lower than that of Example 4 in which the negative to positive pulsed voltage was applied. From the above results, it was revealed that, when alternatingly applying a negative pulsed voltage and a positive pulsed voltage to the bubble generating electrode, it is preferable to first apply a negative pulsed voltage to the bubble generating electrode if conditions other than the voltage are the same.

### [Example 6]

A bubble ejecting apparatus was produced and a bubble ejection method was performed in the same procedure as that in Example 5 except that tungsten was used as a material of the bubble generating electrode, 2 mM KCl instead of 0.5×PBS was used, the positive voltage was +1000 V, the negative voltage was -1000 V, and each pulse width of the positive voltage and the negative voltage was 2 µs. The arrow of FIG. 8 (1) indicates a voltage change applied to the bubble generating electrode, FIG. 8 (2) is a photograph of the tip part of the bubble generating electrode before the voltage is applied, and FIG. 8 (3) is a photograph of the tip part of the bubble generating electrode after the voltage is applied.

Even when the positive to negative pulsed voltage was continuously applied to the bubble generating electrode as illustrated in FIG. 8 (1), ejection of a large number of bubbles from the tip part of the bubble generating electrode was observed as indicated by the arrow in FIG. 8 (3). From the results of Examples 3 to 6 and Comparative example 1, it was found that (1) when applying a negative pulsed voltage and a positive pulsed voltage alternatingly to the bubble generating electrode, it is preferable to first apply the negative pulse voltage to the bubble generating electrode if conditions other than the voltage are the same, (2) it is possible to eject a bubble from the bubble generating electrode even when continuously applying the positive to negative pulsed voltage to the bubble generating electrode by adjusting the material or the shape of the bubble generating electrode, the pulse width, the absolute value of the voltage, or the like, and (3), that is, application of a negative voltage to the bubble generating electrode enables ejection of a bubble.

### [Example 7]

Next, a bubble ejecting apparatus was produced and a bubble ejection method was performed in the same procedure as that in Example 3 except that the tip of the bubble generating electrode was polished into a planar shape, and the applied voltage was 1200 V. The arrow of FIG. 9 (1) indicates a voltage change applied to the bubble generating electrode, FIG. 9 (2) is a photograph of the tip part of the bubble generating electrode before the voltage is applied, and FIG. 9 (3) is a photograph of the tip part of the bubble generating electrode after the voltage is applied in which the portion pointed by the arrow indicates ejected bubbles.

When a negative pulsed voltage was applied to the bubble generating electrode as illustrated in FIG. 9 (1), radial ejection of bubbles was observed as illustrated by the arrow of FIG. 9 (3). It is considered that this is because the electric field concentrates in the tip part in a circumferential shape due to the planar shape of the tip part of the bubble generating electrode. From the above result, it was revealed that, when a negative pulsed voltage is applied to the bubble generating electrode, bubbles are ejected from a part in which the electric field of the bubble generating electrode is likely to concentrate.

### [Example 8]

Next, a bubble ejecting apparatus was produced and a bubble ejection method was performed in the same procedure as that in Example 3 except that the tip of the bubble generating electrode was polished such that the sectional shape was a right triangle, and the applied voltage was 1150 V. The arrow of FIG. 10A (1) indicates a voltage change applied to the bubble generating electrode, FIG. 10A (2) is a photograph of the tip part of the bubble generating electrode before the voltage is applied, and FIG. 10A (3) is a photograph of the tip part of the bubble generating electrode after the voltage is applied in which the portion pointed by the arrow indicates ejected bubbles.

### [Comparative Example 2]

A bubble ejecting apparatus was produced and a bubble ejection method was performed in the same procedure as that in Example 8 except that the power supply device was altered so as to apply a positive voltage. The arrow of FIG. 10B (1) indicates a voltage change applied to the bubble generating electrode, FIG. 10B (2) is a photograph of the tip part of the bubble generating electrode before the voltage is applied, and FIG. 10B (3) is a photograph of the tip part of the bubble generating electrode after the voltage is applied.

When the negative pulsed voltage was applied to the bubble generating electrode as illustrated in FIG. 10A (1), ejection of bubbles from a sharp-pointed portion of the tip part was observed as indicated by the arrow of FIG. 10A (3) as with Example 3. On the other hand, when the positive pulsed voltage was applied to the bubble generating electrode as illustrated in FIG. 10B (1), no bubble was generated at the tip part of the bubble generating electrode as illustrated in FIG. 10B (3). From the above results, it was revealed that ejection of a bubble requires application of a negative voltage to the bubble generating electrode and that, when a negative pulsed voltage is applied to the bubble generating electrode, a bubble is ejected from a part in which the electric field of the bubble generating electrode is likely to concentrate.

### [Example 9]

Next, stainless was used for the material of the bubble generating electrode, and the bubble generating electrode in which the tip was polished so as to be sharper than that in Examples 3, 7, and 8 was produced. Then, a bubble ejecting apparatus was produced and a bubble ejection method was performed in the same procedure as that in Example 3 except that the applied voltage was 1200 V. The arrow of FIG. 11 (1) indicates a voltage change applied to the bubble generating electrode, FIG. 11 (2) is a photograph of the tip part of the bubble generating electrode before the voltage is applied, and FIG. 11 (3) is a photograph of the tip part of the bubble generating electrode after the voltage is applied in which the portion pointed by the arrow indicates ejected bubbles.

When the bubble generating electrode illustrated in Example 9 was used, it was observed that directional continuous bubbles were ejected from the tip part of the bubble generating electrode as is clear from comparison between FIG. 11 (3) (Example 9), FIG. 6A (3) (Example 3), FIG. 9 (3) (Example 7), and FIG. 10A (3) (Example 8). It is considered that this is because, since the tip part was more sharply pointed than that in other Examples, the electric field was likely to concentrate. From the above results, it was revealed that, since the electric field concentrates in a sharp-pointed portion, the number of sharp-pointed parts and the degree of sharpness of the tip part may be adjusted in accordance with the purpose of ejection of bubbles.

### [Comparison between Case of Continuously Applying Negative Pulsed Voltage and Case of Continuously Applying Set of Negative to Positive Pulsed Voltage]

### [Example 10]

Next, in Example 10, a bubble generating electrode produced by polishing the tip by using stainless was used, and a pulsed voltage was applied in the same procedure as that in Example 3. Note that photographs were taken at 800 fps by the high-speed camera.

### [Example 11]

Photographs were taken in the same procedure as that in Example 10 except that a voltage of +1000 V was applied to the bubble generating electrode continuously from a pulsed voltage of -1000 V.

Next, the frames of the high-speed camera in Example 10 and Example 11 were analyzed to analyze the positions of bubbles ejected to the farthest after 1.25 ms and 2.5 ms from application of a negative pulsed voltage. The upper part in FIG. 12 represents analyzed photographs of Example 10, the lower part represents analyzed photographs of Example 11, and each of the dashed lines in the photographs represents the position reached by the ejected bubble. As is clear from the photographs of FIG. 12, the bubble of Example 11 in which a positive voltage was applied continuously from a pulsed voltage of -1000 V reaches farther than the bubble of Example 10 in which a pulsed voltage of -1000 V was applied. The pulse width of the negative voltage was 3 µs, and the pulse width of the subsequently applied positive voltage was 3 µs. Therefore, it can be said that, since 1.25 ms and 2.5 ms are the time by which sufficient time has passed from the initial application of the pulsed voltage, the difference in the position reached by the bubble was due to the fact that the difference in the applied pulsed voltage affected the bubble ejection power. From the above results, it was revealed that the bubble ejection power is stronger when a positive voltage is applied continuously from a negative pulsed voltage to the bubble generating electrode.

### [Study of Principle of Ejection of Bubble]

### [Example 12]

First, a thin wire having a diameter of approximately 0.1 mm was produced from a material of an alloy of platinum and iridium (PTIR, platinum : iridium = 9 : 1), and the tip part was polished to produce bubble generating electrode. Next, a bubble ejection method was performed in the same procedure as that in Example 3 except that a pulsed voltage having a voltage of -600 V and a pulse width of 2 µs was applied.

### [Comparative Example 3]

A bubble ejection method was performed in the same procedure as that in Example 12 except that a pulsed voltage of +600 V was applied.

FIG. 13A is a photograph of the tip part of the bubble generating electrode after the voltage was applied in Example 12. FIG. 13B is a photograph of the tip part of the bubble generating electrode after the voltage was applied in Comparative example 3. As indicated by white circles of FIG. 13A and FIG. 13B, generation of a water flow was observed in both Example 12 and Comparative example 3. Furthermore, generation of bubbles was also observed in a portion surrounded by a dashed line of FIG. 13A. From the above results, it is considered that (a) a bubble is generated only when a negative voltage is applied, (b) a water flow is generated when the voltage changes to relatively the positive direction as indicated by the black arrows of FIG. 13A and FIG. 13B, and (c) a bubble is ejected from the tip part of the bubble generating electrode due to the generated water flow. This is supported by the fact that the bubble of Example 11 (the lower part of FIG. 12), in which a positive voltage was applied continuously from a negative pulsed voltage, in other words, a voltage change rate in relatively the positive direction was larger, was ejected farther than the bubble of Example 10 (in the upper part of FIG. 12, a negative voltage pulse was applied).

### [Industrial Applicability]

The bubble generating electrode 2 used for a bubble ejection method disclosed in the present specification can be easily produced, because the conductive material thereof is exposed. Therefore, the invention is useful in fields in which a processing target is processed by bubbles, such as a field of livestock, agriculture, forestry, and fisheries.

### [List of References]

1 bubble ejecting apparatus
2 bubble generating electrode
3 power supply device
4 counter electrode
5 electric cable
21 tip part
31 control unit
B bubble
L conductor

## Claims

1. A bubble ejection method into a conductor, the bubble ejection method comprising steps of:
contacting a bubble generating electrode to the conductor;
contacting a counter electrode to the conductor or a processing target; and
applying a negative voltage to the bubble generating electrode,
wherein a conductive material is exposed on at least a tip part of the bubble generating electrode.

2. The bubble ejection method according to claim 1, wherein the step of applying the negative voltage to the bubble generating electrode
applies a pulsed voltage, and
starts application to the bubble generating electrode from a negative voltage.

3. The bubble ejection method according to claim 2 further comprising a step of:
applying a positive voltage continuously to the step of applying the negative voltage to the bubble generating electrode.

4. The bubble ejection method according to claim 1,
wherein the step of applying the negative voltage to the bubble generating electrode applies an alternating voltage, and
wherein the alternating voltage has an amplitude on a negative voltage side.

5. The bubble ejection method according to any one of claims 1 to 4, wherein a part other than the tip part, on which the conductive material is exposed, of the bubble generating electrode is covered with an insulating material.

6. The bubble ejection method according to any one of claims 1 to 5, wherein a surface area of the bubble generating electrode in contact with the conductor is smaller than a surface area of the counter electrode in contact with the conductor or a processing target.

7. The bubble ejection method according to any one of claims 1 to 6, wherein the tip part of the bubble generating electrode has a sharp-pointed shape.

8. A power supply device comprising a control unit used for controlling a voltage to be applied,
wherein the control unit performs control so as to start application from a negative pulsed voltage.

9. A power supply device comprising a control unit used for controlling a voltage to be applied,
wherein the control unit performs control so as to apply an alternating voltage having an amplitude on a negative voltage side.

10. A bubble ejecting apparatus comprising at least a bubble generating electrode and the power supply device according to claim 8 or 9.
